# EUROPEAN PATENT APPLICATION

(11) **EP 1 475 105 A1**
(43) Date of publication of application: **10.11.2004**
(21) Application number: 03011721.2
(22) Date of filing: 23.05.2003
(51) Int. Cl.: A61K 51/04

(54) **Bone localising radiopharmaceutical and tubulin-interacting compound combinatorial radiotherapy**

(30) Priority: 09.05.2003 US 469007 P
(71) Applicant: Schering AG, 13342 Berlin (DE)
(72) Inventor: Braendle, Edgar, Sparta, NJ 07871 (US); Hausman, Diana, Seattle, WA 98102 (US)
(74) Representative: Krauss, Jan

(57) **Abstract**

The present invention relates to a method for the improved treatment of a cancerous disease in a patient and/or for the palliation of pain associated with cancer diseases, comprising the administration of a tubulin interacting compound in combination with a bone-localising radiopharmaceutical to said patient in an effective amount that will not cause any substantial ablation of the bone marrow of said patent. In particular, the cancerous disease is selected from the group of cancer diseases, comprising multiple myeloma, leukaemia, lymphoma, breast cancer, prostate cancer, gynecologic cancer, gastric cancer ovarian cancer, lung cancer and/or renal cell carcinoma. In a preferred embodiment, the bone-localising radiopharmaceutical is samarium Sm 153 lexidronam and the tubulin interacting compound is docetaxel.

## Description

The present invention relates to a method for the improved treatment of a cancerous disease in a patient and/or for the palliation of pain associated with cancer diseases, comprising the administration of a tubulin interacting compound in combination with a bone-localising radiopharmaceutical to said patient in an effective amount that will not cause any substantial ablation of the bone marrow of said patent. In particular, the cancerous disease is selected from the group of cancer diseases, comprising multiple myeloma, leukaemia, lymphoma, breast cancer, prostate cancer, gynecologic cancer, gastric cancer ovarian cancer, lung cancer and/or renal cell carcinoma. The invention furthermore relates to the use of a bone-localising radiopharmaceutical for the preparation of a pharmaceutical composition for the treatment of cancer diseases and/or for the palliation of pain associated with cancer diseases, comprising the administration of the bone-localising agent in combination with a tubulin interacting compound to the patient in an effective amount that will not cause a any substantial ablation of the bone marrow of said patient. The invention still further relates to the use of a tubulin interacting compound for the preparation of a pharmaceutical composition for the treatment of cancer diseases and/or for the palliation of pain associated with cancer diseases, comprising the administration of the tubulin interacting compound in combination with a bone-localising radiopharmaceutical to the patient in an effective amount that will not cause any substantial ablation of the bone marrow of said patient. In a preferred embodiment, the bone-localising radiopharmaceutical is samarium Sm 153 lexidronam and the tubulin interacting compound is docetaxel.

### Background of the invention

Prostate cancer is the most common noncutaneous malignancy in men and is the second leading cause of cancer death in males in the United States. There will be an estimated 189,000 new cases diagnosed and 30,200 deaths due to prostate cancer in 2002 (Ahmedin 2002). In patients with advanced prostate cancer, the primary site of metastasis is bone, although soft-tissue metastases can also occur. Because of the hormone-responsive nature of prostate cancer, the initial management of patients with advanced disease usually involves androgen deprivation. While androgen deprivation leads to stabilisation or regression of disease in more than 80% of patients, the median duration of response is less than 2 years due to the eventual overgrowth of hormone-refractory cells. Following development of hormone-refractory prostate cancer (HRPC), median survival is approximately 1 year (Kish 2001). Treatment options for patients with HRPC are limited and have been focused on palliation, highlighting the need for more effective therapeutic strategies.

Until recently, the primary therapeutic goals for HRPC patients were symptomatic relief and quality of life improvement, with the hope of a modest survival benefit. The combination of mitoxantrone and prednisone was approved by the FDA based solely on its palliative effects, without evidence of prolongation in overall survival. However, recent studies suggest that cytotoxic agents that target intracellular microtubules have more pronounced activity against HRPC than previously believed.

Several estramustine-based regimens have recently demonstrated the ability to reduce PSA levels and induce measurable disease responses in patients with HRPC (Hudes 1997a, 1997b, 1999, Seidman 1992, Petrylak 1999a, 1999b, Kreis 1999a, 1999b, Savarese 2001). The docetaxel/estramustine combination has been viewed as the most attractive of these regimens because of its clinical efficacy, convenient administration schedule, and improved toxicity profile. The regimen has demonstrated evidence of synergistic antitumor activity against HRPC in both preclinical and clinical studies (Petrylak 1999a, Petrylak 1999b, Kreis 1999a, Kreis 1999b, Savarese 1999).

Docetaxel inhibits tumour growth through induction of microtubule stabilisation by binding to B-tubulin and promotion of bcl-2 inactivation, thereby sensitising malignant cells to apoptotic stimuli (Ringel 1991, Haldar 1997, Friedland 1999). Estramustine, an estradiol-nitrogen mustard conjugate, also exerts antitumor effects by binding with B-tubulin and microtubule-associated proteins (Laing 1997). In Phase 2 studies, docetaxel administered IV every 3 weeks plus daily oral estramustine (for 1 to 21 consecutive days per cycle) produced substantial clinical activity in patients with HRPC (45% to 82% of patients had > 50% decrease in PSA) (Petrylak 1999a, Petrylak 1999b, Kreis 1999a, Kreis 1999b, Savarese 2001, Sinibaldi 2002, Scholz 1998). However, the combination was associated with significant nausea, diarrhoea, neutropenia, cumulative fluid retention, and an increased risk for thrombotic events.

The incidence of Grade 3/4 neutropenia ranged from 40% to 75% in these studies. More recent phase 1/2 studies of weekly docetaxel plus shorter courses of oral estramustine have demonstrated comparable efficacy with less toxicity in the HRPC setting, with markedly decreased incidence of neutropenia (3%-12% Grade 3 neutropenia, 0%- 4% Grade 4 neutropenia) (Natale 1998, Natale 1999, Kosty 2000, Copur 2001).

Samarium Sm 153 lexidronam (Quadramet®) is a radiopharmaceutical that has an affinity for skeletal tissue and concentrates in areas of bone turnover secondary to invasion by tumour (Goeckeler 1987). It has been approved by the FDA for the relief of pain in patients with osteoblastic metastatic bone lesions at a dose of 1.0 mCi/kg. The radioisotope, with a half-life of 46.3 hours, emits a 103 keV gamma ray for external imaging and a number of beta particles (average energy 233 keV) for localised radiotherapy. The range of emission of samarium Sm 153 lexidronam is only 1.7 mm in bone, limiting the exposure of bone marrow and other adjacent tissues to radiation. The degree of skeletal uptake of samarium Sm 153 lexidronam correlates with the extent of osteoblastic bone disease. The ratio of uptake of samarium Sm 153 lexidronam by skeletal lesions relative to normal bone is approximately 5:1. There is no significant uptake by nonosseous tissue (Eary 1993). Clearance of samarium Sm 153 lexidronam is exclusively renal, with the majority of excretion occurring during the first 8 hours after administration, and complete excretion by 12 hours (Serafini 2001).

Similar to strontium 89 chloride, therapy with samarium Sm 153 lexidronam results in symptomatic relief of bone pain in approximately 80% of treated patients. However, because of the shorter half-life of samarium Sm 153 lexidronam, a higher dose of radioactivity can be delivered, resulting in a higher biologic effect and more rapid onset of action (Serafini 2001). In this Phase 3 study, two-thirds of patients in the 1.0 mCi/kg samarium Sm 153 lexidronam dose group who had pain relief at Week 4 still had pain relief at Week 16 (Serafini 2001).

The major toxicity of samarium Sm 153 lexidronam is haematologic. In pooled data from three Phase 3 controlled studies at a dose of 1.0 mCi/kg, a 40% to 50% reduction in leukocyte and platelet counts was seen, with the nadir occurring at a median of 4 weeks, and recovery generally complete by 5-8 weeks. Grade 3/4 haematologic toxicity was seen in < 10% of patients, and generally occurred in patients who had recently undergone external beam radiation or chemotherapy, had low blood counts on study entry, or had proven or probable bone marrow involvement with metastatic prostate cancer (Serafini 2001). In a Phase 1/2 study, repeated dosing with 1.0 mCi/kg samarium Sm 153 lexidronam was not associated with cumulative toxicity (Bayouth 1994). In this study, WBC and platelet nadir values were no lower after multiple doses than after the initial dose, and results showed that 1.0 mCi/kg doses could be repeated every 6 to 10 weeks. Other Phase 1/2 studies also found that repeated dosing with 1.5 to 2.0 mCi/kg samarium Sm 153 lexidronam for up to three and four cycles was feasible (Alberts 1997; data on file, Berlex).

A number of chemotherapy agents have demonstrated radiosensitising properties, including docetaxel and estramustine (Pradier 2001, Koukourakis 1998a, 1998b, Koukourakis 1999, Ekiov 1994, Ryu 1994, Edgren 2000, Kim 1994), making them attractive candidates for use in combination with radionuclides. The majority of radionuclide combination experience is with strontium 89 chloride. Estramustine has been evaluated in combination with strontium 89 chloride in several trials because of its radiosensitising properties and lack of myelosuppression. The combination has been shown to be well tolerated, with no Grade 3/4 neutropenia, 6% Grade 3 thrombocytopenia, and 3% Grade 3 anaemia. PSA response rates were in the order of 31 %, with a median duration of 7 months (Dahut 1998). Similar results have been observed when strontium 89 chloride was combined with carboplatin (Sciuto 1998).

More extensive work has been done with the combination of strontium 89 chloride and doxorubicin. Tu et al. combined weekly doxorubicin with strontium 89 chloride and observed a 76% overall response rate. In addition, there was an improved survival (median 15.4 months) and a PSA decline of> 75% in one third of patients (Tu 1997). In this trial, 4% of patients experienced Grade 3 thrombocytopenia, with no occurrence of Grade 4 thrombocytopenia. The incidence of Grade 3 and 4 neutropenia was 28% and 16%, respectively. Tu et al. then conducted a Phase 2 trial in which 72 HRPC patients with bone metastases were randomized to receive weekly doxorubicin alone or doxorubicin plus strontium 89 chloride. In this trial, there was a statistically significant improvement in overall survival (27.7 months for combined therapy vs 16.8 months for chemotherapy alone) (Tu 2001). The incidence of Grade 3/4 neutropenia was 44% in patients who received doxorubicin plus strontium compared to 22% in patients who received doxorubicin alone.

Similar results were observed in another trial evaluating the same type of regimen (Hatfield 1999). Strontium 89 chloride and vinblastine/estramustine have been given concurrently to HRPC patients (Akerley 2002). In this study, 48% of patients had a > 50% PSA decline.

There was a 23% incidence of Grade 3/4 neutropenia and 20% incidence of Grade 3/4 thrombocytopenia. The median overall survival of 13 months in this trial compares favourably with historical data.

The experience of samarium Sm 153 lexidronam combined with chemotherapy agents is more limited. There are no published reports or abstracts involving the combination of samarium Sm 153 lexidronam and docetaxel. Samarium Sm 153 lexidronam has been evaluated in combination with doxorubicin or mitomycin with bolus fluorouracil in one study (Turner 2001). Turner et al. reported an overall response rate of 75%, with 25% of patients having complete resolution of pain. In 15 of 34 treated patients, there was radiographic and bone scan evidence of regression of skeletal metastases. Dose-limiting toxicity was reversible myelosuppression consisting predominantly of delayed thrombocytopenia.

Recent studies of weekly docetaxel plus shorter courses of estramustine have demonstrated similar efficacy with less toxicity in the HRPC setting compared to less frequent, higher dose docetaxel/estramustine therapy (Natale 1998, Natale 1999, Kosty 2000, Copur 2001). In addition, these agents have also been shown to have radiosensitising properties (Pradier 2001, Koukourakis 1998, Koukourakis 1999, Ekiov 1994, Ryu 1994, Edgren 2000, Kirn 1994). Samarium Sm-153 lexidronam is a radiopharmaceutical that selectively targets bone lesions, allowing for delivery of therapy to this area of high-volume disease in patients with HRPC.

WO 00/76556 discloses the use of ¹⁵³Sm-EDTMP together with bioactive agents, such as antineoplastic chemotherapeutic agents for the partial or complete suppression of bone marrow and the treatment of bone marrow diseases, such as cancer. One example would be paclitaxel. The chemotherapeutic agent can be applied in conjunction with the radiotherapeutic. The publication is directed to replacing TBI (total bone irradiation) with the administration of, e.g. ¹⁶⁶Ho-DOTMP in a bone-associated pathology in a dosage to deliver 20 to 60 Gy to the patient, and the administration of the chemotherapeutic after the complex. The document is therefore related to the suppression of the bone marrow in order to provide a "background" for bone marrow transplantation. In general, the publication is directed to the most effective killing of the diseased bone marrow of the patient. Nothing is disclosed with respect to pain relief as indication.

Arteaga de Murphy C, et al. ("Labelling of Re-ABP with 188Re for bone pain palliation." Appl Radiat Isot 2001 Mar;54(3):435-42) disclose etidronate and medronate that have been labelled with technetium-99m (99mTc-HEDP, 99mTc-MDP) for bone scanning and with rhenium-188 (188Re-HEDP) to palliate the pain resulting from bone metastases. Furthermore, alendronate, ABP, a new bisphosphonate, was labelled with SnF2-reduced-188Re for bone pain palliation.

Silberstein (Silberstein EB. Systemic radiopharmaceutical therapy of painful osteoblastic metastases. Semin Radiat Oncol 2000 Jul;10(3):240-9) discloses that bone pain from osteoblastic metastases can be ameliorated 40% to 80% of the time and the efficacy of radiopharmaceuticals containing phosphorus 32, strontium 89, samarium 153, rhenium 186, and tin 117m in a treatment that is repeated at about 9- to 12-week intervals, perhaps earlier with (153)Sm lexidronam, (186)Re etidronate, and (117m)Sn pentetate. The duration of action of pain reduction ranges from 2 weeks to many months.

Despite several attempts, still no efficient and effective treatment showing less side effects for the patients based on a bone-localising radiopharmaceutical, such as, for example ¹⁵³Sm-EDTMP or ¹⁶⁶Ho-DOTMP, together with a chemotherapeutical agent has been found. The present invention addresses this problem.

It is therefore an object of the present invention, to provide an improved treatment based on a bone-localising radiopharmaceutical together with a chemotherapeutical agent with the ability of the independently effective treatment modalities to demonstrate both safety and clinical synergy when used as a combination regimen. It is a further object of the present invention, to provide means for such improved therapy.

### Description of the invention

This first object of the present invention is solved by a method for the improved treatment of a cancerous disease in a patient and/or for the palliation of pain associated with cancer diseases, comprising the administration of a tubulin interacting compound in combination with a bone-localising radiopharmaceutical to said patient in an effective amount that will not cause any substantial ablation of the bone marrow of said patent.

As used herein, the term "substantial is generally to be understood by reference to the art-recognised definitions. When used with respect to ablation of the bone marrow of a patient, with "substantial" is meant an ablation, wherein the damage to the bone marrow as caused by the radiopharmaceutical, will cause an ablation of the hematologically active cells (including the stem cells) inside the bone marrow to such an extent that the patient will require a bone-marrow supportive treatment in order to maintain/retain a sufficient function of the bone marrow. One example of a "substantial" ablation will be a complete ablation of the bone marrow of the patient.

Another aspect of the present invention provides for the use of a bone-localising radiopharmaceutical for the preparation of a pharmaceutical composition for the treatment of cancer diseases and/or for the palliation of pain associated with cancer diseases, comprising the administration of the bone-localising agent in combination with a tubulin interacting compound to the patient in an effective amount that will not cause a any substantial ablation of the bone marrow of said patient. According to another aspect of the present invention, the invention provides for the use of a tubulin interacting compound for the preparation of a pharmaceutical composition for the treatment of cancer diseases and/or for the palliation of pain associated with cancer diseases, comprising the administration of the tubulin interacting compound in combination with a bone-localising radiopharmaceutical to the patient in an effective amount that will not cause any substantial ablation of the bone marrow of said patient.

According to another aspect of the method according to the present invention, said cancerous disease is selected from the group of cancerous diseases, comprising multiple myeloma, leukaemia, lymphoma, breast cancer, ovarian cancer, prostate cancer, lung cancer, and renal cell carcinoma. Furthermore, said cancerous disease can be a cancer with bone metastasis, such as metastatic breast cancer or metastatic prostate cancer. Most preferred is a method according to the present invention, wherein said cancerous disease is hormone-refractory prostate cancer (HRPC).

As one part of the treatment according to the present invention, said bone-localising radiopharmaceutical is selected from the group consisting of the radionuclides ¹⁷⁷Lu, ¹⁷⁵Yb, ¹⁸⁶Re, ¹⁸⁸Re, ⁹⁰Y, ¹¹⁷mSn, ¹⁵³Sm, ¹⁶⁶Ho or ¹⁵⁹Gd (Comment: not all of these nuclide are used in the indication for bone seeking agents) Please delete all of those that will not make sense that are complexed with a bone-localising chelating agent selected from the group consisting of aminophosphonic acids, in particular samarium Sm 153 lexidronam, and ⁸⁹Sr-chloride. Preferred are aminophosphonic acids which are selected from the group consisting of EDTMP, DOTMP, DTPMP, HEEDTMP, NTMP, HEDP, HMDP, MDP, and TTHMP, and combinations thereof.

According to yet another aspect of the present invention, said bone-localising radiopharmaceutical is administered at a dose of between about 0.005 to 5.0 mCi/kg, and preferably administered at a dose of 1.0 mCi/kg. In a preferred method according to the present invention, said tubulin interacting compound is administered before or after the administration of said bone-localising radiopharmaceutical. Preferably, the amount that is given is effective for bone pain relief in said patient. Preferably, said tubulin interacting compound is administered at a dose of between 20 to 100 mg/m²/week, and preferably administered at a dose of between 30 to 70 mg/m²/week. Most preferred are a weekly administration of Taxol of approx. 70 mg/m² and of Taxotere of approx. 25-40 mg/m²/week. For other anti tubulin agents the actual regime and the dose can slightly differ. Nevertheless, these slight differences can be easily determined by the attending physician, based on the actual status and individual clinical parameters of the patient to be treated.

As the other part of the treatment according to the present invention, said tubulin interacting compound is a chemotherapeutic agent selected from the group comprising docetaxel, estramustine, doxorubicin, paclitaxel, tamoxifen, vinblastine, vinorelbine, vindesine, vincristine cyclophosphamide, and the like. In particular, anti-microtubule agents are as follows: Vinca alkaloids (vincristine, vinblastine, vindesine, vinorelbine), Taxanes (paclitaxel, docetaxel), estramustine phosphate, and compounds like, for example, epothilone A and B. Other suitable tubulin interacting compounds can be found in the respective literature, for example, in Jiang JD, et al. "Double blockade of cell cycle at g(1)-s transition and m phase by 3-iodoacetamido benzoyl ethyl ester, a new type of tubulin ligand." Cancer Res. 2002 Nov 1;62(21):6080-8.; Bocca C, et al. "Cytoskeleton-interacting activity of geiparvarin, diethylstilbestrol and conjugates." Chem Biol Interact. 2001 Sep 28;137(3):285-305.; Botta B, et al. "Aryltetralin lignans: chemistry, pharmacology and biotransformations." Curr Med Chem. 2001 Sep;B(11):1363-81.; Poncet J. "The dolastatins, a family of promising antineoplastic agents." Curr Pharm Des. 1999 Mar;5(3):139-62.; D'Amato RJ, et al. "2-Methoxyestradiol, an endogenous mammalian metabolite, inhibits tubulin polymerization by interacting at the colchicine site." Proc Natl Acad Sci U S A. 1994 Apr 26;91(9):3964-8.; Kuo SC, et al. "Synthesis and cytotoxicity of 1,6,7,8-substituted 2-(4'-substituted phenyl)-4-quinolones and related compounds: identification as antimitotic agents interacting with tubulin." J Med Chem. 1993 Apr 30;36(9):1146-56, and Hendriks HR, et al. "Preclinical antitumour activity and animal toxicology studies of rhizoxin, a novel tubulin-interacting agent." Ann Oncol. 1992 Nov;3(9):755-63.

Another important aspect of the method according to the present invention relates to the "timing" (i.e. treatment regimen) of the dosages of the radiotherapeutic and/or the tubulin interacting compound to be applied. Preferably, the administration of said bone-localising radiopharmaceutical is performed in a time period ranging from about 1 to 30 days, and preferably 1 to 7 days, before and/or after the administration of said tubulin interacting compound. Preferred is a method according to the present invention. wherein the bone-localising radiopharmaceutical is administered in at least 8 week-intervals during the course of a weekly administration of said tubulin interacting compound. Alternatively, the bone-localising radiopharmaceutical is administered in at least 8 week-intervals during the course of an administration every three weeks of said tubulin interacting compound.

In one particular example, samarium Sm 153 lexidronam (Quadramet®) will be administered at a dose of 1.0 mCi/kg on Day 2 of Cycle 1 (i.e., once every 16 weeks; one cycle = 8 weeks). Each patient will receive up to two doses of Samarium Sm 153 lexidronam if tolerated. Samarium Sm 153 lexidronam will be administered through an established intravenous (i.v.) line over a period of 1 minute. Docetaxel (Taxotere®) will be administered i.v. over 1 hour each week at a dose of 30 mg/m²/week on Day 1 of Weeks 1-3 and 5-7 of each 8- week cycle.

Preferred is a method according to the present invention, wherein said bone-localising radiopharmaceutical and said tubulin interacting compound are administered in 16 week treatment cycles, said bone-localising radiopharmaceutical being administered during week 1 of said cycles, and said tubulin interacting compound being administered during weeks 1-3, 5-7, 9-11, and 13-15 of said treatment cycles. (Be aware that we are at a very early stage of clinical development and that all these timeframes are very critical and potentially could change. Furthermore for other chemotherapeutic agents and for other bone seeking agents the time schedule would be totally different.) (This seems to be a filing-yes-or-no situation. If we wait for the final result of the clinical trials, that could hurt our position. I'd say one should take the risk of the present uncertainty and maybe file add-ons later (by CIP's and/or folow up applications. Otherwise, we must delay the filing until the final results are there. Currently, I do not see chances to fix this "moving target" further.)

Further preferred is a method according to the present invention, wherein said bone-localising radiopharmaceutical is administered on day 2 of said treatment cycles, and said tubulin interacting compound is administered on day 1 of weeks 1-3, 5-7, 9-11, and 13-15 of said treatment cycles, more preferred is a method, wherein said bone-localising radiopharmaceutical is 153Sm-EDTMP being administered at a dose of 1mCi/kg, and said tubulin interacting compound is docetaxel being administered at a dose of 30mg/m²/week.

According to yet another embodiment of the method according to the present invention, in addition estramustine phosphate sodium is administered during weeks 1-3, 5-7, 9-11, and 13-15 of said treatment cycles. In accordance with the above, for example, estramustine phosphate sodium (Emcyt®) will be administered on Days 1-3 of Weeks 1-3 and 5-7 of each 8-week cycle and will be given at a dose of between approx. 100 to 400 mg, preferably approx. 280 mg twice a day orally (p.o.). Even more preferably, said estramustine phosphate sodium is administered on days 1 to 3 of weeks 1-3, 5-7, 9-11, and 13-15 of said treatment cycles. Ideally, said estramustine phosphate sodium is administered at a dose of 280 mg twice a day. Of course, the dose can be varied, depending from the individually tolerated dose and the amount that would be needed in order to enhance the treatment effect.

In one method according to the present invention, 1 to 8 of said treatment cycles are performed. The treatment regimen can also been summarised in table 1 as follows.

**Table 1:**

| First exemplary treatment regimen according to the present invention | | | | | | | | |
|---|---|---|---|---|---|---|---|---|
| Week | 1 | 2 | 3 | 4 | 5 | 6 | 7 | 8 |
| radiopharmaceutical | + | - | - | - | - | - | - | - |
| Tubulin interact. comp. | + | + | + | - | + | + | + | - |
| estramustine (opt.) | + | + | + | - | + | + | + | - |

| week | 9 | 10 | 11 | 12 | 13 | 14 | 15 | 16 |
|---|---|---|---|---|---|---|---|---|
| radiopharmaceutical | - | - | - | - | - | - | - | - |
| Tubulin interact. comp. | + | + | + | - | + | + | + | - |
| estramustine (opt.) | + | + | + | - | + | + | + | - |

Preferred is also method according to the present invention, wherein said bone-localising radiopharmaceutical and said tubulin interacting compound are administered in 8 week treatment cycles, said bone-localising radiopharmaceutical being administered during week 1 of said cycles, and said tubulin interacting compound being administered during weeks 1-3, and 5-7 of said treatment cycles. Preferred is a method according to the present invention, wherein said bone-localising radiopharmaceutical is administered on day 2 of said treatment cycles, and said tubulin interacting compound is administered on day 1 of weeks 1-3, and 5-7 of said treatment cycles.

Further preferred is a method according to the present invention, wherein said bone-localising radiopharmaceutical is 153Sm-EDTMP being administered at a dose of approx. 1mCi/kg, and said tubulin interacting compound is docetaxel being administered at a dose of administered at a dose of between 30 to 70 mg/m²/week.

According to yet another method according to the present invention, in addition estramustine phosphate sodium is administered during weeks 1-3, and 5-7 of said treatment cycles. Even more preferably, said estramustine phosphate sodium is administered on days 1 to 3 of weeks 1-3, and 5-7 of said treatment cycles. Ideally, said estramustine phosphate sodium is administered at a dose of 280 mg twice a day.

In one method according to the present invention, 1 to 16 of said treatment cycles are performed. The above treatment regimen can also been summarised in table 2 as follows.

**Table 2:**

| Second exemplary treatment regimen according to the present invention | | | | | | | | |
|---|---|---|---|---|---|---|---|---|
| Week | 1 | 2 | 3 | 4 | 5 | 6 | 7 | 8 |
| Radiopharmaceutical | + | - | - | - | - | - | - | - |
| Tubulin interact. Comp. | + | + | + | - | + | + | + | - |
| estramustine (opt.) | + | + | + | - | + | + | + | - |

According to the method according to the present invention, the administration is intravenous (i.v.), orally (p.o.), and/or subcutaneously. Further, the administration can be in a bolus dose or in several doses, independently from the different embodiments of the method according to the present invention.

Finally, according to yet another embodiment of the present invention, the different treatment regimen can be combined, e.g. first performing the second ("short") exemplary treatment regimen according to the present invention and, thereafter, performing the first ("long") exemplary treatment regimen according to the present invention, or vice versa.

Another aspect of the present invention is related to a method for the treatment of a bone-associated cancer in a patient and/or for the palliation of pain associated with a bone-associated cancer, comprising the administration of a tubulin interacting compound in combination with a bone-localising radiopharmaceutical to said patient in an effective amount that will not cause any substantial ablation of the bone marrow of said patient. In general, said bone-associated cancer is selected from the group of metastatic cancerous diseases, comprising multiple myeloma, leukaemia, lymphoma, breast cancer, ovarian cancer, prostate cancer, lung cancer, and renal cell carcinoma. Most preferred is a method according to the present invention, wherein said cancerous disease is hormone-refractory prostate cancer (HRPC).

As one embodiment of the method of treatment of a bone-associated cancer according to the present invention, said tubulin interacting compound is administered before or after the administration of said bone-localising radiopharmaceutical, preferably in an amount that is effective for bone pain relief in said patient.

As one part of the treatment of a bone-associated cancer according to the present invention, said bone-localising radiopharmaceutical is selected from the group consisting of the radionuclides ¹⁷⁷Lu, ¹⁷⁵Yb, ¹⁸⁶Re, ¹⁸⁸Re, ⁹⁰Y, ¹¹⁷mSn, ¹⁵³Sm, ¹⁶⁶Ho or ¹⁵⁹Gd that are complexed with a bone-localising chelating agent selected from the group consisting of aminophosphonic acids, in particular samarium Sm 153 lexidronam, and ⁸⁹Sr-chloride. Preferred are aminophosphonic acids which are selected from the group consisting of EDTMP, DOTMP, DTPMP, HEEDTMP, NTMP, HEDP, HMDP, MDP, and TTHMP, and combinations thereof.

According to yet another aspect of the method for treatment of a bone-associated cancer of the present invention, said bone-localising radiopharmaceutical is administered at a dose of between about 0.005 to 5.0 mCi/kg, and preferably administered at a dose of 1.0 mCi/kg. In a preferred method for treatment of a bone-associated cancer according to the present invention, said tubulin interacting compound is administered at a dose of between 20 to 100 mg/m²/week, further preferably administered at a dose of between 30 to 70 mg/m²/week, and most preferred administered at a dose of approximately 30 mg/m²/week.

As the other part of the method for treatment of a bone-associated cancer according to the present invention, said tubulin interacting compound is a chemotherapetic agent selected from the group comprising docetaxel, estramustine, doxorubicin, paclitaxel, tamoxifen, vinblastine, vinorelbine, vindesine, vincristine cyclophosphamide, and the like. Other suitable compounds are indicated above.

Another important aspect of the method for treatment of a bone-associated cancer according to the present invention relates to the "timing" (i.e. treatment regimen) of the dosages of the radiotherapeutic and/or the tubulin interacting compound to be applied. In general, the treatment schedule can be as above with minor amendments that are based on the different s of bone-associated cancer that might be involved and the individual patient parameters of the patient(s) to be treated. These amendments can be easily determined by the skilled person, such as the attending physician.

Yet another aspect of the present invention relates to a kit of parts for the treatment of a cancer disease and/or for the palliation of pain associated with cancer diseases, said kit comprising the following components: a) a pharmaceutically effective amount of a bone-localising radiopharmaceutical, b) a pharmaceutically effective amount of a tubulin interacting compound, and c) optionally, suitable pharmaceutically acceptable buffers and/or diluents, wherein the amount of the bone-localising radiopharmaceutical comprises an effective amount of said bone-localising radiopharmaceutical that will not cause any substantial ablation of the bone marrow when applied to a patient.

Preferred is a kit according to the present invention, wherein said bone-localising radiopharmaceutical is selected from the group consisting of the radionuclides ¹⁷⁷Lu, ¹⁷⁵Yb, ¹⁸⁶Re, ¹⁸⁸Re, ⁹⁰Y, ¹¹⁷mSn, ¹⁵³Sm, ¹⁶⁶Ho or ¹⁵⁹Gd that are complexed with a bone-localising chelating agent selected from the group consisting of aminophosphonic acids, in particular samarium Sm 153 lexidronam, and ⁸⁹Sr-chloride. Preferably, said aminophosphonic acids are selected from the group consisting of EDTMP, DOTMP, DTPMP, HEEDTMP, NTMP, HEDP, HMDP, MDP, and TTHMP, and combinations thereof.

According to one embodiment of the kit according to the present invention, said bone-localising radiopharmaceutical is suitable for the administration at a dose of between about 0.005 to 5.0 mCi/kg, preferably at a dose of 1.0 mCi/kg, and said tubulin interacting compound is suitable for the administration at a dose of between 20 to 100 mg/m²/week, further preferably administered at a dose of between 30 to 70 mg/m²/week, and most preferred administered at a dose of approximately 30 mg/m²/week.

According to yet another embodiment of the kit according to the present invention, the tubulin interacting compound is suitable for administration before or after the administration of the bone-localising radiopharmaceutical, preferably suitable for administration at a dose of between 20 to 100 mg/m²/week, further preferably administered at a dose of between 30 to 70 mg/m²/week, and most preferred administered at a dose of approximately 30 mg/m²/week.

According to yet another embodiment of the kit according to the present invention, the tubulin interacting compound is a directly and/or indirectly tubulin-interacting chemotherapetic agent selected from the group comprising docetaxel, doxorubicin, tamoxifen, vinblastine, vinorelbine, vindesine, vincristine cyclophosphamide, and the like. Other suitable tubulin interacting compounds are as above. As an additional aspect of the present invention, other chemotherapeutical agents, such as, for example, paclitaxel and/or estramustine can be administered during the course of the therapy as well. Preferably, said bone-localising radiopharmaceutical is suitable for administration in a time period ranging from about 1 to 30 days, and preferably 1 to 7 days, before and/or after the administration of said tubulin interacting compound. Even more preferably, said bone-localising radiopharmaceutical is suitable for administration in at least 8 week-intervals during the course of a weekly administration of said tubulin interacting compound. Even more preferably, said bone-localising radiopharmaceutical is suitable for administration in at least 8 week-intervals during the course of an administration every three weeks of said tubulin interacting compound.

According to the present invention, the kits can either be ready-to-use or form kits that are used to prepare the active agents immediately before use, for example, by mixing different ingredients. One example for such a kit is described for tumour and metastasis scintigraphy in Chauhan UP, et al. (in: "Evaluation of a DMSA kit for instant preparation of 99mTc(V)-DMSA for tumour and metastasis scintigraphy." Int J Rad Appl Instrum B 1992 Nov;19(8):825-30) for instantly preparing 99mTc(V)-DMSA. In another aspect, the kit can be produced by a robot, as described, for example, in Luurtsema G, et al ("An automated synthesis module for preparation of L-3-[123I]iodo-alpha-methyl tyrosine." Appl Radiat Isot 2001 Dec;55(6):783-8).

"Tubulin interacting compound" shall mean a chemotherapeutic agent that directly (e.g. by interacting with the tubulin stem) and/or indirectly (e.g. by interaction with the microtubular associated proteins (MAP) or interference with the genetic expression of parts of the microtubular machinery of the cell) interferes with the tubulin-system of the cell(s) in order to disturb said system directly and/or indirectly, such as an inhibitor of cell division acting on tubulin.

"Treatment" or "therapy" shall mean a process of the reversing or ameliorating or reducing a condition of a certain type or certain types of cells or tissues with respect to an abnormal behaviour, such as a proliferative disease. Preferably, a treatment is applied to a patient. The treatment according to the present invention is performed in "treatment cycles" which shall mean either 8-week or 16-week time periods.

A "bone-localising radiopharmaceutical" shall mean a radiopharmaceutical that is specifically used for the therapy and/or pain palliation of diseases related with bone-pain and/or bone-disease. Such diseases are either directly located in the bone itself (e.g. cancers of the bone marrow) or indirectly related to bone, such as bone-metastatic cancerous diseases, comprising multiple myeloma, leukaemia, lymphoma, breast cancer, ovarian cancer, prostate cancer, lung cancer, gynecologic cancer, gastric cancer and renal cell carcinoma, in particular hormone-refractory prostate cancer (HRPC).

In "combination with" shall mean the administration of the tubulin interacting compound and/or the radiopharmaceutical and/or a chemotherapeutic agent according to the present invention to the patient in need thereof in a manner that allows for a synergistic therapeutical effect (radiosynergy) of said combination. The combination can be either concomitantly or spaced apart in time. As an example, in the course of the present invention, the radiopharmaceutical is administered at week 1 of the treatment cycle, in combination with the tubulin interacting compound, and, optionally, with the chemotherapeutic agent. Nevertheless, any combination within a treatment cycle will be regarded as in "combination with" each other.

"Pharmaceutical composition" shall mean a composition comprising at least one active ingredient, whereby the composition is amenable to investigation for a specified, efficacious outcome in a mammal (for example, and not limitation, a human). Those of ordinary skill in the art will understand and appreciate the techniques appropriate for determining whether an active ingredient has a desired efficacious outcome based upon the needs of the artisan, in this case the treatment of bone-associated diseases, such as cancer.

In the course of the present invention, a study was performed in order to evaluate and confirm the safety and tolerability of combination therapy according to the present invention. Patients with hormone-refractory adenocarcinoma of the prostate with more than one osteoblastic bone metastasis confirmed by technetium (⁹⁹Tc)-labelled bisphosphonates and eligible for chemotherapy treatment were enrolled in the study.

All publications as cited herein are incorporated herein by reference in their entirety. The invention shall now be further described based on the following example, without being limited thereto.

### Examples

In order to confirm the primary objective of the study, the safety and tolerability of the combination of samarium Sm153 lexidronam and docetaxel therapy, optionally together with estramustine, will be studied. As a secondary objective, the efficacy of combination samarium Sm153 lexidronam and docetaxel therapy, optionally together with estramustine treatment, in patients with HRPC will be studied.

Part 1 of the study will evaluate the safety and tolerability of a single 8-week cycle of combination samarium Sm 153 lexidronam, estramustine, and docetaxel therapy in a small cohort of patients (N = approx. 10).

Part 2 will confirm the safety and tolerability of samarium Sm 153 lexidronam given once every 16 weeks (total of two doses) in combination with estramustine/docetaxel therapy in a larger cohort of patients (N = approx. 60) treated for a longer period of time (32 weeks). In addition, the study will assess the efficacy of combination samarium Sm 153 lexidronam, (optionally estramustine), and docetaxel treatment in patients with HRPC.

This will be a conventional Phase 1/2 open-label two-part study. The design of samarium Sm 153 lexidronam combined with weekly administration of 30 mg/m²/week docetaxel and, optionally, 560 mg/day estramustine administered 3 days/week will be chosen because it is expected to allow closer monitoring of safety and efficacy and is expected to have a lower overall toxicity profile compared to the regimen of docetaxel administered once every 3 weeks plus estramustine administered 5 days a week. Control groups will not be used in the present study.

Methods of assigning patients to treatment groups are not used, as this will be an open-label study with no randomisation.

### Study design

### Selection of study population

Patients with hormone-refractory adenocarcinoma of the prostate with more than one osteoblastic bone metastasis confirmed by ⁹⁹Tc-labeled bisphosphonates and eligible for chemotherapy treatment will be included. Unless defined otherwise in the protocol, an individual patient will be only included once in the study.

### Inclusion Criteria

Patients must meet the following criteria to enter the study:
- Histologically documented adenocarcinoma of the prostate.
- Presence of more than one osteoblastic bone metastasis confirmed by ⁹⁹Tc-labeled bisphosphonates on bone scan.
- Clinically progressive hormone-refractory disease, as documented by one or more of the following: Two documented consecutive increases in PSA over a previous reference value (first increase at least 1 week after reference value). Increased PSA level must be at least 5 ng/mL. New lesions on bone scan (progressive measurable disease). Increase in bidimensionally measurable disease.
- If no prior surgical castration, serum testosterone must be < 50 ng/mL with continuation of gonadotropin-releasing hormone agonist.
- Patients receiving antiandrogen treatment must demonstrate no decrease in PSA values on at least two occasions at least 1 week apart after antiandrogen withdrawal. Flutamide must be discontinued for at least 4 weeks, and bicalutamide, megastrol acetate, cyproterone acetate, or nilutamide must be discontinued for at least 6 weeks prior to study entry.
- If receiving bisphosphonate therapy, agreement to hold bisphosphonates 2 weeks before and 2 weeks after samarium Sm 153 lexidronam dosing.
- Age ≥ 18 years.
- Karnofsky performance status of ≥70%.
- Life expectancy of at least 8 months.
- Patients with lower urinary tract obstruction or incontinence must consent to catheterisation of the bladder for up to 6 hours after administration of samarium Sm 153 lexidronam.
- Agreement to carry out scheduled clinic visits and laboratory testing as required throughout the study.
- Ability to understand and the willingness to sign a written informed consent document.

### Exclusion criteria

Patients are excluded from the study if they meet any of the following criteria:
Prior chemotherapy; Prior treatment with a systemic radiotherapeutic bone agent; Receipt of any other investigational drugs within 4 weeks of study entry; Receipt of palliative radiotherapy for bone metastases within 90 days of study entry; Pathologic long-bone fractures (unless surgically stabilised), imminent pathologic long-bone fracture (cortical erosion on radiography > 50%), or spinal cord compression; Metastatic involvement of> 75% of ribs, vertebrae, and pelvic bones on bone scan; Known hypersensitivity to phosphonate compounds, drugs formulated with polysorbate 80, or dexamethasone; Known hypersensitivity to estradiol or nitrogen mustard; History of active thrombophlebitis or thromboembolic disorders; History of deep vein thrombosis or pulmonary embolus; Known NYHA class III-IV congestive heart failure; Clinically relevant change in angina within 6 months of study entry; Myocardial infarction within 1 year of study entry; Peripheral neuropathy (≥ Grade 2); Active CNS or epidural brain metastasis; Active or uncontrolled bacterial, viral, or fungal infection; Clinical diagnosis of disseminated intravascular coagulation; ANC < 1500/mm³; WBC count < 3500/mm³; Platelet count < 135,000/mm³; Hemoglobin < 10 g/dL; Serum creatinine > 1.5 mg/dL; Total bilirubin ≥ 1.5 x ULN; SGOT (AST) and SGPT (ALT) ≥ 1.5 x ULN; PT/PTT > 1.5ULN; or PSA < 5 ng/mL.

### Removal of patients from treatment or assessment

Patients are permanently removed from the study if they experience any of the following: DLT as defined below; More than two dose modifications of docetaxel; Inadequate ANC and platelet count status or recovery (ANC ≥ 1500/mm³ and platelet count ≥ 75,000/mm³) after a delay in samarium Sm 153 lexidronam treatment of up to 2 weeks; Grade 2 anaphylaxis or hypersensitivity if there is a recurrence after rechallenge with docetaxel treatment; Symptomatic arrhythmia; Symptomatic pleural effusion; Need for haematopoietic growth factor support; Disease progression; Receipt of antineoplastic therapy other than designated study treatment.

### Treatments

Treatment will be administered on an outpatient basis in the clinical setting. No investigational or commercial agents or therapies other than those described in this protocol are administered with the intent to treat the disease with the exception of continued use of gonadotropin-releasing hormone agonist or bisphosphonates. Patients receiving bisphosphonate therapy will bisphosphonates withheld starting 2 weeks before and 2 weeks after each dose of samarium Sm 153 lexidronam. All patients undergo baseline visits and drug infusion in accordance with package insert guidelines. Clinical and laboratory monitoring will be performed in accordance with package insert guidelines.

### Pharmaceutical compounds

All study medications except samarium Sm 153 lexidronam are obtained through commercial sources. Samarium Sm 153 lexidronam will be provided by Berlex.

### Samarium Sm 153 lexidronam

Samarium Sm 153 lexidronam used will be manufactured for Berlex Laboratories (Richmond, CA) by DuPont Pharmaceuticals Co. (Billerica, MA) under license from Cytogen Corp. (Princeton, NJ). Quadramet® is supplied frozen in a single-dose 10 mL glass vial containing 1850 ±185 MBq/mL (50 ± 5 mCi/mL) of samarium-153, at calibration. The vial is shipped in a lead shield. The drug product expires 48 hours after the time of calibration noted on the label, or 8 hours after thawing, whichever is earlier. In accordance with package insert requirements, Quadramet® must be stored frozen at -10° to -20°C in a lead shielded container. Storage and disposal of Quadramet® should be controlled in a manner that complies with the appropriate regulations of the government agency authorised to license the use of this radionuclide.

### Estramustine Phosphate Sodium

Estramustine Phosphate Sodium (Emcyt® (estramustine)) is manufactured for Pharmacia & Upjohn Company (Kalamazoo, MI) by Pharmacia & Upjohn S.p.A. (Ascoli Piceno, Italy). Emcyt® is supplied as white opaque capsules, each containing estramustine phosphate sodium as the disodium salt monohydrate equivalent to 140 mg estramustine phosphate. In accordance with package insert requirements, Emcyt® capsules should be stored between 2°C and 8°C (36°F to 46°F).

### Docetaxel

Docetaxel; Taxotere® (docetaxel) is manufactured by Aventis Pharmaceuticals Products Inc. (Bridgewater, NJ). Taxotere® is supplied in a single-dose vial as a sterile, pyrogen-free, nonaqueous, viscous solution with an accompanying sterile, nonpyrogenic, diluent (13% ethanol in Water for Injection) vial.

In accordance with package insert requirements, Taxotere® should be stored between 2°C and 25°C (36°F and 77°F). Retain in the original package to protect from bright light. Freezing does not adversely affect the product. Procedures for proper handling and disposal of anticancer drugs should be considered.

### Statistical Methods

Response rates are estimated using the observed proportion of responders of those considered evaluable for response. To quantify sampling error in the observed response rate, we will construct upper one-sided confidence intervals (CIs) of the response rate by using the normal approximation to the binomial distribution. A patient's survival time will be defined as the time between enrollment into the study and last date of contact whether or not death occurred with censoring of data as necessary. The survival distribution of the time to death will be estimated using the product-limit method of Kaplan and Meier.

Endpoints for this study are analysed descriptively based on summary statistics (mean, median, frequency, etc.). Primary and secondary endpoints will be calculated and described. The 2-sided chi-square test will be the only statistical testing performed. Patients are enrolled over a period of up to 12 months (including follow-up).

A total of approx. 60 patients will be enrolled in the study, with each site enrolling 1 to 20 patients. It is anticipated that this number results in approx. 50 evaluable patients who will have completed two cycles of treatment. A sample size of approx. 50 patients will be sufficient to test the hypothesis that the proportion of patients with DLTs is 0.20 (20%) versus null hypothesis of 0.40 (40%) with 90% power using a 2-sided chi-square test and a significance level of a = 0.05.

### Treatment Part 1:

An initial cohort of approx. 10 patients will receive combination treatment. If two patients in the cohort exhibit a DLT, another four patients are treated at that dose with no more than two experiencing a DLT before Part 2 of the study will be initiated. The study will be permanently stopped if five or more of the initial 10 patients experience a DLT. Patients who do not experience a DLT in Part 1 can continue on to Part 2.

### Treatment Part 2:

Patients carried over from Part 1 who do not experience DLT receive up to three additional cycles of combination therapy. All other patients enrolled in Part 2 receive up to four cycles of treatment. This will be stopped if the cumulative incidence of DLT exceeds 40% at any time.

### Primary endpoints of the study

Primary endpoints are the proportion of patients experiencing a dose-limiting toxicity (DLT) in treatment cycles 1 and 2 (cumulative through Week 16). DLT is defined by Grade 4 neutropenia (absolute neutrophil count [ANC] < 500/mm³) with fever > 38.3°C, Grade 4 thrombocytopenia (platelet count < 10,000/mm³), or any nonhaematologic Grade 3 or 4 toxicity using National Cancer Institute (NCI) Common Toxicity Criteria. Grade 3 or 4 nausea, vomiting, mucositis, fatigue, or alopecia, or Grade 3 diarrhoea will be not be considered DLT events.

### Secondary endpoints

Secondary endpoints are, survival, response rate (as measured by prostate-specific antigen [PSA], computerised tomography [CT] scan or magnetic resonance imaging [MRI], or bone scan), and disease progression (as measured by PSA, CT scan or MRI, or bone scan).

Safety in this trial will be monitored on a continuous basis, and any DLTs reported to the study management within 24 hours of their occurrence.

### Study objectives

### Primary objective

The primary objective will be to establish the safety and tolerability based on the incidence and severity of adverse events associated with samarium Sm 153 lexidronam administered in combination with optional estramustine and docetaxel.

### Safety and Tolerability of One Cycle/Four Cycles of Combination Treatment

For safety, approx. 10 patients will be treated with the two- to three-drug regimen and evaluated for safety and tolerability. Patients will receive 1.0 mCi/kg samarium Sm 153 lexidronam on Day 2 of the 8-week cycle. Estramustine will be optionally administered on Days 1-3 of Weeks 1-3 and 5-7 and will be given at a dose of 280 mg twice a day PO. Docetaxel will be administered at 30 mg/m²/week on Day 1 of Weeks 1-3 and 5-7 of the 8-week cycle.

All patients who do not experience DLT and have stable or responding disease with treatment during the first cycle enter into Part 2 (see above) and will receive up to three additional cycles of combination treatment.

Cycles 1 and 3 consist of combination samarium Sm 153 lexidronam/opt. estramustine/docetaxel therapy, and Cycles 2 and 4 consist of combination estramustine/docetaxel therapy. Patients must meet eligibility criteria prior to starting an additional cycle. Patients are permanently discontinued from the study if they experience disease progression. Study stopping rules are outlined above.

Total duration of the study for each patient will be up to 36 weeks. This consists of four 8-week cycles of treatment (32 weeks total) and a follow-up evaluation 4 weeks after completion of the final cycle. Additional follow-up evaluations for survival status continue for up to 12 months after completion of the final cycle.

### Secondary objectives

Secondary objectives are to evaluate the efficacy of the combination regimen(s), including its impact on survival, response rate, disease progression, and pain.

### Actual treatments

Samarium Sm 153 lexidronam will be administered through an established i.v. line over a period of 1 minute once every two 8-week treatment cycles (i.e., 16 weeks). Each patient receives up to two doses of samarium Sm 153 lexidronam if tolerated. Refer to the package insert for a general description of the drug and its formulation, preparation, administration, storage, stability, and adverse events.

Samarium Sm 153 lexidronam is sent frozen directly to the study site. Samarium Sm 153 lexidronam must be stored frozen in a shielded container until administration. After thawing and prior to administration, the investigator assays samarium Sm 153 lexidronam for total radioactivity using a radioisotope dose calibrator with the proper calibration setting for samarium Sm 153 lexidronam. The total activity administered to each patient will be determined by assaying the syringe containing the dose both prior to and immediately following administration of samarium Sm 153 lexidronam. An aliquot of samarium Sm 153 lexidronam will be assayed to ensure the pH is in the range of 7.0-8.5.

Special precautions should be taken following samarium Sm 153 lexidronam administration to minimise the risk of radioactive contamination of clothing, bed linen, and the patient's environment. Patients with urinary tract obstruction or urinary incontinence should be catheterised for 6 hours after administration of samarium Sm 153 lexidronam. Investigators must follow Nuclear Regulatory Commission (NRC) regulations regarding the discharge of patients from the study site

Samarium Sm 153 lexidronam is contraindicated in patients who have known hypersensitivity to EDTMP or similar phosphonic acid chelators. If signs of acute toxicity occur during the administration of samarium Sm 153 lexidronam, the infusion should be stopped and appropriate supportive measures should be taken.

### Estramustine

Estramustine will be administered on Days 1-3 of Weeks 1-3 and 5-7 of each 8-week cycle and will be given at a dose of 280 mg twice a day p.o.. Refer to the package insert for a general description of the drug and its formulation, preparation, administration, storage, stability, and adverse events. Patients were requested to return unused estramustine to the study site.

All patients receive concomitant warfarin for prophylaxis of deep vein thrombosis. Warfarin will be administered at a dose of 2 mg/day p.o. every day for the duration of the study and will be started on the same day as estramustine administration.

### Docetaxel

Docetaxel will be administered i.v. at a dose of 30 mg/m²/week on Day 1 of Weeks 1-3 and 5-7 of each 8-week cycle. Each patient receives up to a total of four cycles of docetaxel. Refer to the package insert for a general description of the drug and its formulation, preparation, administration, storage, stability, and adverse events.

Patients will receive dexamethasone for prophylaxis of hypersensitivity reaction and fluid retention. Dexamethasone will be administered at a dose of 8 mg p.o. 12 hours prior to docetaxel dose, 1 hour prior to docetaxel dose, and 12 hours after docetaxel dose.

### Precautions

Myelosuppression has been observed with both samarium Sm 153 lexidronam and docetaxel. Although the risk of Grade 3/4 neutropenia appears to be decreased when docetaxel is given weekly at low doses rather than once every 3 weeks at high doses, patients should be monitored closely for signs and symptoms of myelosuppression on a weekly and as-needed basis throughout the entire study period.

### Selection of doses in the study

The doses of estramustine and docetaxel for this study will be selected based on recent studies of weekly low-dose docetaxel plus shorter courses of estramustine that demonstrated similar efficacy with less toxicity in the HRPC setting compared to less frequent, higher dose docetaxel/estramustine therapy (Natale 1998, Natale 1999, Kosty 2000, Copur 2001). In addition, these agents have also demonstrated radiosensitising properties (Pradier 2001, Koukourakis 1998, Koukourakis 1999, Ekiov 1994, Ryu 1994, Edgren 2000, Kirn 1994). The FDA-approved dose of 1.0 mCi/kg samarium Sm 153 lexidronam will be selected to be given once every 16 weeks as this interval has been shown to allow for complete recovery of cell counts as well as provide maximum duration of pain relief (Serafini 2001).

### Selection and timing of dose for each patient

Patients will receive 1.0 mCi/kg samarium Sm 153 lexidronam on Day 2 of the 8-week cycle (dosing will be permitted between Days 1 and 3 to allow for possible drug shipment/scheduling conflicts). Estramustine will be optionally administered on Days 1-3 of Weeks 1-3 and 5-7 and will be given at a dose of 280 mg twice a day p.o.. Docetaxel will be administered at 30 mg/m²/week on Day 1 of Weeks 1-3 and 5-7 of the 8-week cycle.

Patients will be instructed to administer estramustine (as well as prophylactic dexamethasone and warfarin) at the same times each day and to record the date and time of each administration in the patient diary in addition to analgesic use, other concomitant medications, pain, and adverse events.

### Definition of DLT

DLT will be defined as any of the following: Grade 4 neutropenia (ANC < 500/mm³) with fever > 38.3°C; Grade 4 thrombocytopenia (platelet count < 10,000/mm³); Any nonhaematologic Grade 3 or 4 toxicity with the exception of nausea, vomiting, mucositis, fatigue, or alopecia, or Grade 3 diarrhoea using NCI CTC criteria (Version 2.0, April 30, 1999). Disease progression or lack of disease response are not be considered DLT events.

### Interruption, dose reduction, or discontinuation due to toxicity

If a patient experiences toxicity not meeting the definition for DLT, the following criteria will be used to delay or modify samarium Sm 153 lexidronam, estramustine, and docetaxel dosing or to permanently discontinue a patient from the study.

### Samarium Sm 153 Lexidronam: Haematological Toxicity

For the second dose samarium Sm 153 lexidronam treatment (Day 2 of Cycle 3), patients must have ANC ≥ 1500/mm³, platelet count ≥ 75,000/mm³, and satisfactory recovery from non-haematological toxicity. If these criteria are not met on the scheduled day of treatment, the treatment must be delayed for up to 2 weeks. If ANC and platelet count do not recover within 2 weeks, the patient will be discontinued from the study.

In addition, the patient's clinical and haematological responses to the initial dose of samarium Sm 153 lexidronam are taken into consideration prior to repeat treatment with samarium Sm 153 lexidronam.

### Estramustine: Hepatic Toxicity

If hepatic toxicity occurred (bilirubin or SGOT/SGPT > 1.5 ULN), estramustine is reduced or the patient is permanently discontinued from the trial at the discretion of the treating physician. Bilirubin and SGOT/SGPT values are obtained prior to each cycle of therapy and reviewed by the treating physician.

### Docetaxel: Haematological Toxicity

If ANC is < 1500/mm³ prior to beginning a cycle of treatment, treatment will be delayed until ANC is ≥ 1500/mm³. Docetaxel will be resumed at 100% dose unless the delay is > 7 days, in which case, decrease the subsequent docetaxel dose by 25%. If ANC does not recover within 2 weeks of delay in therapy, the patient will be discontinued from the study. If platelet count is < 75,000/mm³ on the day of therapy, treatment will be delayed until ≥ 75,000/mm³ and subsequent doses of docetaxel will be permanently reduced by 25%. If the platelet count does not recover within 2 weeks of delay in therapy, the patient will be discontinued from the study. If a patient experiences a bleeding episode with a platelet nadir of < 40,000/mm³ for longer than 7 days, docetaxel will be reduced by 25%. If the bleeding episode does not resolve with platelet count ≥ 75,000/mm³ within 2 weeks of delay in therapy, the patient will be discontinued from the study.

If a patient experiences similar haematological toxicity after a dose reduction, a further 25% reduction in subsequent doses will be implemented. Up to two dose reductions in docetaxel therapy will be permitted. If a patient requires further dose reduction due to hematological toxicity, study treatment should be discontinued.

### Docetaxel: Nonhaematological Toxicity

If a patient experiences nonhaematological toxicity, the following dose modification criteria are used: Patients who experience Grade 3 or 4 fatigue will have treatment withheld until they attain ≤ Grade 2 recovery. Subsequent docetaxel doses are reduced by 25%. If ≤ Grade 2 recovery will be not attained within 2 weeks of delay in therapy, the patient will be discontinued from the study; Patients who experience Grade 3 diarrhoea or mucositis, or Grade 2 neuropathy (see below) have docetaxel reduced by 25%; Docetaxel will be reduced by 25% at the discretion of the treating physician if a patient appeared to be poorly tolerating study drug treatment; Anaphylaxis/Hypersensitivity: For moderate (Grade 2) symptoms: if moderate symptoms recur after rechallenge, docetaxel infusion will be stopped, the patient will be discontinued from study treatment, and the event will be reported as an adverse event. If a patient experiences severe life-threatening symptoms (Grade 3 or 4), study treatment will be discontinued; Cardiovascular: Symptomatic arrhythmia: stop chemotherapy and discontinue the patient from study treatment, Clinical congestive heart failure: if a patient experienced Grade 3 or 4 LVEF according to NCI CTC criteria, the patient will be discontinued from the study; Fluid Retention: Fluid retention is a recognised complication of docetaxel that may be ameliorated by diuretics. Loop diuretics should be used judiciously to treat fluid retention; Patients are discontinued from the study only if they experience symptomatic pleural effusion; Peripheral Neuropathy: If Grade 1 intensity, continued therapy at the full dose; If Grade 2 intensity, reduce docetaxel by 25%; Up to two dose reductions in docetaxel therapy are permitted for peripheral neuropathy. If a patient required further dose reduction due to neuropathy, study treatment will be discontinued. Hepatic: If hepatic toxicity occurs (bilirubin or SGOT/SGPT > 1.5 ULN), docetaxel will be reduced or the patient will be permanently discontinued from the trial at the discretion of the treating physician. Bilirubin and SGOT/SGPT values are obtained prior to each cycle of therapy and reviewed by the treating physician.

### Prior and concomitant therapy

Patients receiving LH-RH agonists continue the primary androgen ablation. Patients continue on medications for symptom relief (e.g., analgesics, hypnotics, anxiolytics) or an as-needed basis. Patients requiring other antineoplastic therapy following administration of study treatment will be removed from the trial.

Haematopoietic growth factor support will not allowed be while patients are enrolled on the study. The use of erythropoietin will be at the discretion of the treating physician. Patients requiring growth factor support will be discontinued from the study but will be followed for safety. All medications administered during the study shall be reported on the CRFs and in the source documents.

### Treatment compliance

Patients will be supplied a diary in which to record the date and time of each estramustine, dexamethasone, and warfarin dose in addition to analgesic use, other concomitant medications, pain, and adverse events. Study site staff reviews the diary with the patient at each scheduled clinic visit and transcribes all pertinent information onto the CRF. The diaries are retained at the study site as part of the source documentation.

### Safety and efficacy variables

Procedures for safety and efficacy measurements and flow chart. The investigative team at each study site will be responsible for performing all evaluations and recording information on source documents as well as completing CRFs. When possible, the same staff personnel performs all evaluations for a given patient. All laboratory analyses are performed by local laboratories except for serum PSA analyses, which are performed at a central laboratory.

The beginning of each treatment cycle will be designated as Day 1. Every attempt will be made to schedule clinic visits on the day stipulated for evaluation. All visits had to be conducted within 7 days of the stipulated evaluation day. Study-mandated blood tests are drawn within 48 hours of study drug administration. Evaluations that are performed are described in the following section and summarised in the Schedule of Evaluations (Table 1, below).

### Samarium Sm 153 lexidronam procedures

The following procedures apply to administration of samarium Sm 153 lexidronam treatment only (Cycles 1 and 3):
Prior to administration of samarium Sm 153 lexidronam treatment:
   1) Give the patient at least 500 mL of i.v. or oral fluids within 4 hours prior to treatment.
   2) Catheterise the patient if necessary.
   3) Administer samarium Sm 153 lexidronam in accordance with appropriate procedures and precautions stipulated in the package inserts and at the dose specified in this protocol. Flush the agent remaining in the syringe, needle, and i.v. tubing with normal saline.

After administration of samarium Sm 153 lexidronam treatment:
1) Record vital signs every 30 minutes for the first 2 hours after dosing. If signs of acute toxicity occur during administration of the agent, the infusion should be stopped and appropriate supportive measures taken. In case of acute toxicity, consideration should be given to the development of hypocalcemia or an allergic reaction to EDTMP.
2) Prior to discharge from the clinic, have the patient ingest at least 500 mL of fluids.
3) Obtain a radiation exposure reading at a distance of 1 meter from the patient immediately following dosing and prior to discharge from the clinic.
4) The treatment room will be surveyed by the Radiation Safety Officer. Patients may be discharged from the test facility when the exposure reading complies with state and federal regulations.

### End-of-study evaluations

Patients will have the following procedures completed at the end of the study:
Patient diary review; VAS; Adverse event assessment; Physical examination, including weight; Vital signs; CBC; Serum chemistry (creatinine, BUN, total bilirubin, alkaline phosphatase, SGOT, SGPT, sodium, potassium, chloride, bicarbonate, glucose, albumin, total protein, calcium, phosphorus); Serum PSA (analysed at a central laboratory); Coagulation profile (PT/PTT); Urinalysis; Karnofsky performance status; 99Tc bone scan; CT scan or MRI for patients with measurable disease.

### 28-day follow-up evaluations:

Patients will have the following procedures completed during the last study visit (>. 28 days after the last dose of study drug):
Patient diary review; VAS; Adverse event assessment; Physical examination, including weight; Vital signs; CBC; Serum chemistry (creatinine, BUN, total bilirubin, alkaline phosphatase, SGOT, SGPT, sodium, potassium, chloride, bicarbonate, glucose, albumin, total protein, calcium, phosphorus); Serum PSA (analysed at a central laboratory); Coagulation profile (PT/PTT); Urinalysis; Karnofsky performance status.

### Long-term follow-up assessments

All patients will be contacted by clinic visit or telephone at 3, 6, 9, and 12 months after the last study visit to determine survival and additional disease-related treatment received.

### Appropriateness of measurements

Prostate-Specific Antigen Working Group (PSAWG) disease assessment criteria (Bubley 1999), VAS (Akhar-Danesh 2001), and Karnofsky performance status are standard accepted tools of measurement in HRPC.

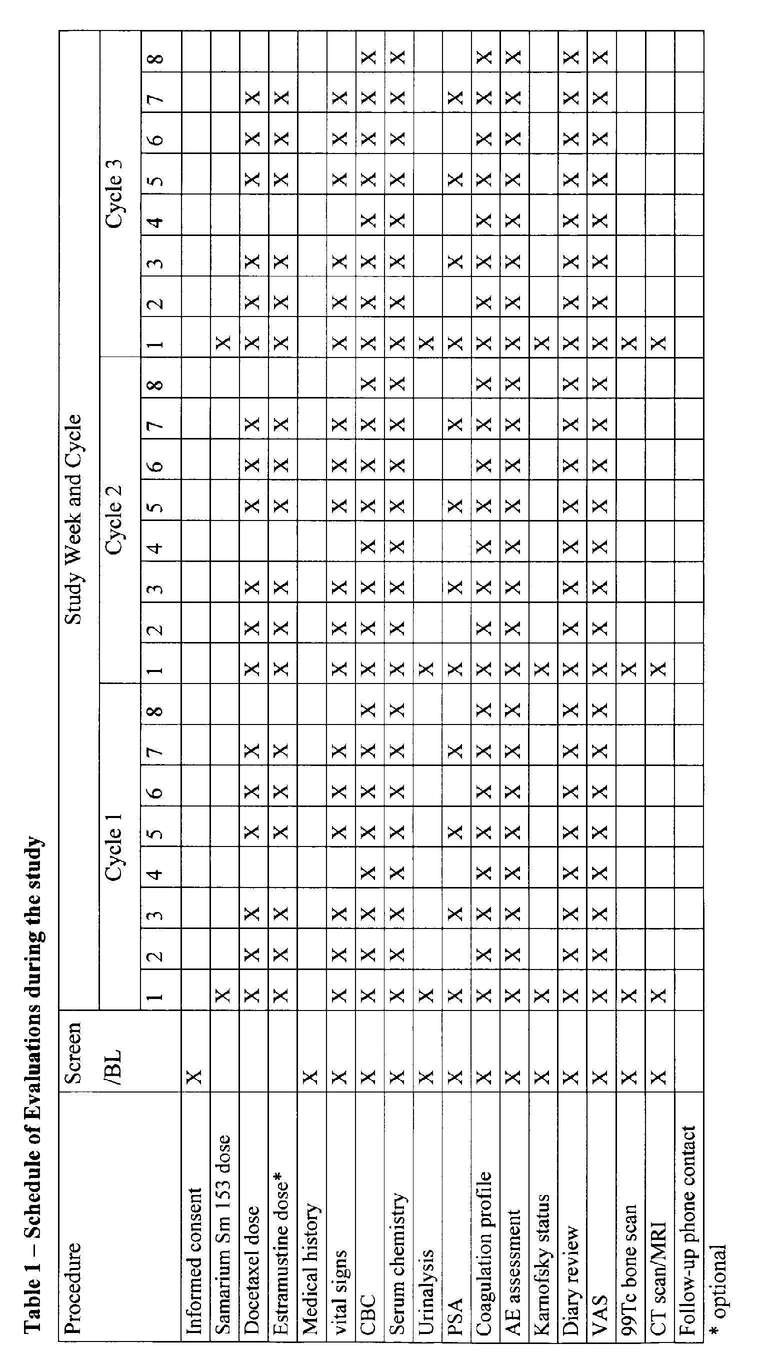

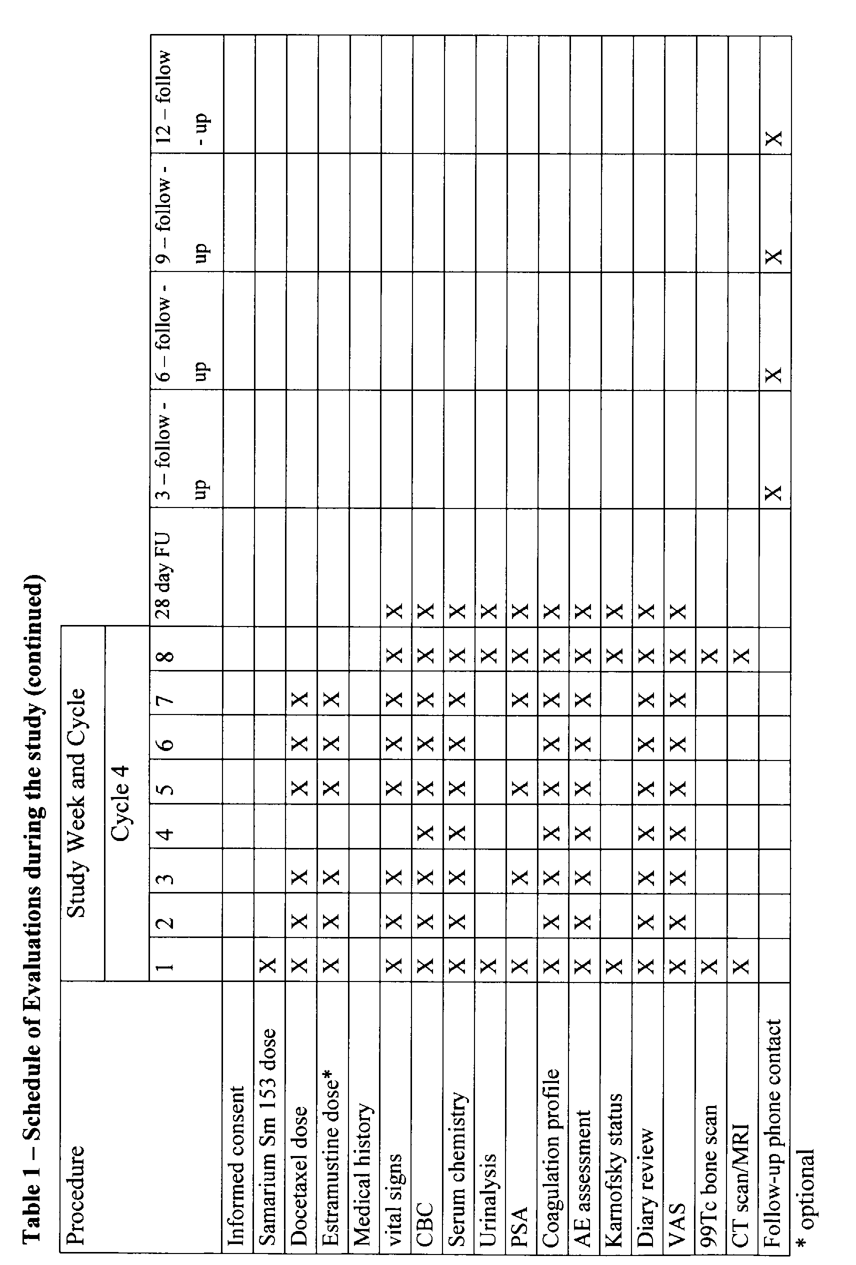

This trial of samarium Sm 153 lexidronam, optionally estramustine, and docetaxel (RED) will demonstrate the feasibility of applying combined modality treatment in the setting of metastatic prostate carcinoma. Although concurrent chemoradiotherapy often is associated with increased toxicity, it is assumed the RED will be well tolerated and easily administered to outpatients. Response rate, duration of response, and survival will confirm the systemic activity of RED, and will suggest a long-lasting and potential synergistic effect at sites of boney disease.

Combination therapy with RED will be quite effective, particularly when evaluated in the context of a multiinstitutional trial (BrUOG: five hospitals with varied patient populations including private, indigent, veterans hospitals), but maybe it will be difficult to estimate the contribution from the individual agents.

Finally, although combined modality therapy will be used, RED will not be associated with enhanced toxicity.

### Reference list

Ahmedin J, Thomas A, Taylor Murray T, et al. Cancer Statistics, 2002. CA Cancer J Clin 2002;52:23-47.

Akerley W, Butera J, Wehbe T, et al. A multi-institutional, concurrent chemoradiation trial of strontium-89, estramustine, and vinblastine for hormone refractory prostate carcinoma involving bone. Cancer 2002;94(6): 1654-60.

Akhtar-Danesh N. A review of statistical methods for analysing pain measurements. Eur J Pain 2001;5(4):457-63.

Alberts AS, Smit BJ, Louw WK, et al. Dose response relationship and multiple dose efficacy and toxicity of samarium-153-EDTMP in metastatic cancer to bone. Radiother Oncol 1997;43(2): 175-9.

Bubley GJ, Carducci M, Dahut W, et al. Eligibility and response guidelines for phase II clinical trials in androgen-independent prostate cancer: recommendations from the Prostate-Specific Antigen Working Group. J Clin Oncol 1999;17(11):3461-7.

Dahut W, et al. Strontium-89 and estramustine: delaying treatment failure in hormone-refractory prostate cancer. Proc Am Soc Clin One 1998;17:329a.

Eary JF, Collins C, Stabin M, et al. Samarium-153-EDTMP biodistribution and dosimetry estimation. J Nucl Med 1993;34(7):1031-6.

Edgren M, Lennemas B. Estramustine a radiosensitising agent. Anticancer Res 2000;20(4):2677-80.

Ekiov S, Westlin JE, Rikner G, et al. Estramustine potentiates the radiation effect in human prostate tumor transplant in nude mice. Prostate 1994;24(1):39-45.

Friedland D, Cohen J, Miller R Jr, et al. A phase II trial ofdocetaxel (Taxotere) in hormone-refractory prostate cancer: correlation ofantitumor effect to phosphorylation ofBcl-2. Semin Oncol 1999;26(5Suppll7):19-23.

Goeckeler WF, Edwards B, Volkert WA, et al. Skeletal localization of samarium-153 chelates: potential therapeutic bone agents. J Nucl Med 1987;28(4):495-504.

Haldar S, Basu A, Croce CM. Bcl2 is the guardian ofmicrotubule integrity. Cancer Res 1997;57(2):229-33.

Hatfreld A. Intermittent alternating chemotherapy/hormone therapy combined with strontium-89 for androgen resistant prostate cancer. Proc Am Soc Clin One 1999; 18.

Hudes G, Einhom L, Ross E, et al. Vinblastine versus vinblastine plus oral estramustine phosphate for patients with hormone-refractory prostate cancer: A Hoosier Oncology Group and Fox Chase Network phase III trial. J Clin Oncol 1999;17(10):3160-6.

Hudes G. Estramustine-based chemotherapy. Semin Urol Oncol 1997a; 15(1): 13-9.

Hudes GR, Nathan F, Khater C, et al. Phase II trial of 96-hour paclitaxel plus oral estramustine phosphate in metastatic hormone-refractory prostate cancer. J Clin Oncol 1997b;15(9):3156-63.

Kim JH, Khil MS, Kim SH, et al. Clinical and biological studies of estramustine phosphate as a novel radiation sensitizer. Int J Radiat Oncol Biol Phys 1994;29(3):555-7.

Kish JA, Bukkapatnam R, Palazzo F. The treatment challenge of hormone-refractory prostate cancer. Cancer Control 2001;8(6):487-95.

Kosty MP, Ferreira A, Bryntesen T, et al. Weekly docetaxel and low dose estramustine phosphate in hormone refractory prostate cancer: a phase II study. Proc Am Soc Clin Oncol 2000;19:365a (abstract).

Koukourakis MI, Bahlitzanakis N, Froudarakis M, et al. Concurrent conventionally fractionated radiotherapy and weekly docetaxel in the treatment of stage IIIb non-small-cell lung carcinoma. Br J Cancer 1999;80(11): 1792-6.

Koukourakis M, et al. Concurrent twice-a-week docetaxel and radiotherapy: A dose escalation trial with immunological toxicity evaluation. J Rad One Biol Phys 1998;43:107-14.

Koukourakis MI, Kourousis C, Kamilaki M, et al. Weekly docetaxel and concomitant boost radiotherapy for non-small cell lung cancer. A phase I/II dose escalation trial. Eur J Cancer 1998;34(6):838-44.

Kreis W, Budman D. Daily oral estramustine and intermittent intravenous docetaxel (Taxotere) as chemotherapeutic treatment for metastatic, hormone-refractory prostate cancer. Semin Oncol 1999a; 26(5 Suppi 17):34-8.

Kreis W, Budman DR, Fetten J, et al. Phase I trial of the combination of daily estramustine phosphate and intermittent docetaxel in patients with metastatic hormone refractory prostate carcinoma. Ann Oncol 1999;10(1):33-8.

Laing N, DahllofB, Hartley-Asp B, et al. Interaction of estramustine with tubulin isotypes. Biochem 1997;36(4):871-8.

Natale RB, Zaretsky S. Phase I/II trial of estramustine (E) with taxotere (T) or vinorelbine (V) in patients (pts) with metastatic hormone-refractory prostate cancer (HRPC). Proc Am Soc Clin Oncol 17:338a, 1998 (abstract).

Natale RB, Zaretsky S. Phase I/II trial of estramustine (E) and taxotere (T) in patients (pts) with metastatic hormone-refractory prostate cancer (HRPC). Proc Am Soc Clin Oncol 18:348a, 1999 (abstract).

Petrylak D. Phase I trial of docetaxel with estramustine in androgen independent prostate cancer. J Clin Oncol 1999a; 17(3):958-67.

Petrylak DP, Macarthur R, O'Connor J, et al. Phase I/II studies of docetaxel (Taxotere) combined with estramustine in men with hormone-refractory prostate cancer. Semin Oncol 1999;26(5 Suppl 17):28-33.

Pradier 0, Rave-Frank M, Lehmann J, et al. Effects of docetaxel in combination with radiation on human head and neck cancer cells (ZMK-1) and cervical squamous cell carcinoma cells (CaSki). Int J Cancer 2001;91(6): 840-5.

Ringel I, Horwitz SB. Studies with RP 56976 (taxotere): a semisynthetic analogue of taxol. J NatI Cancer Inst 1991;83(4):288-91.

Ryu S, Gabel M, Khil MS, et al. Estramustine: a novel radiation enhancer in human carcinoma cells. Int J Radiat Oncol Biol Phys 1994;30(1):99-104.

Savarese D, Taplin ME, Halabi S, et al. A phase II study of docetaxel (Taxotere), estramustine, and low-dose hydrocortisone in men with hormone-refractory prostate cancer: preliminary results of cancer and leukemia group B Trial 9780. Semin Oncol 1999;26(5 Suppi 17):39-44.

Savarese D, et al. Phase II study of docetaxel, estramustine, and low-dose hydrocortisone in men with hormone refractory prostate cancer: a final report ofCALGB 9780. J Clin Oncol 2001;19(9):2509-16.

Scholz M, Strum S, McDermed J. A phase II trial of taxotere (T), estramustine (E) combination in patients (pts) with prostate cancer (PC). Proc Am Soc Clin Oncol 17:342a, 1998 (abstract).

Sciuto R, Festa A, Tofani A, et al. Platinum compounds as radiosensitizers in strontium-89 metabolic radiotherapy. ClinTer 1998;149(921):43-7.

Seidman AD, Scher HI, Petrylak D, et al. Estramustine and vinblastine: use of prostate specific antigen as a clinical trial end point for hormone refractory prostatic cancer. J Urol 1992; 147(3 Pt 2):931-4.

Serafini AN. Systemic metabolic radiotherapy with samarium-153 EDTMP for the treatment of painful bone metastasis. Q J Nucl Med 2001;45(1):91-9.

Sinibaldi VJ, Carducci MA, Moore-Cooper S, et al. Phase II evaluation of docetaxel plus one-day oral estramustine phosphate in the treatment of patients with androgen independent prostate carcinoma. Cancer 2002;94(5): 1457-65.

Copur SM, Ledakis P, Lynch J, et al. Weekly docetaxel and estramustine in patients with hormone-refractory prostate cancer. Semin Oncol 2001 ;28(4 Suppi 15): 16-21.

Tu S, Delpassand E, Jones D, et al. Strontium-89 combined with doxorubicin in the treatment of patients with androgen independent prostate cancer. Urol Oncol 1997;2:191-7.

Tu S, Millikan RE, Mengistu B, et al. Bone targeted therapy for advanced androgen-independent carcinoma of the prostate: a randomized phase II trial. Lancet 2001 ;357(9253):336-41.

Turner JH, Claringbold PG, Martindale AA, et al. Samarium-153 EDTMP and radiosensitizing chemotherapy for treatment of disseminated skeletal metastases. Eur J Nucl Med 1992;16:S125.

## Claims

1. Use of a bone-localising radiopharmaceutical for the preparation of a pharmaceutical composition for the treatment of cancer diseases and/or for the palliation of pain associated with cancer diseases, comprising the administration of the bone-localising radiopharmaceutical agent in combination with a tubulin interacting compound to the patient in an effective amount that will not cause any substantial ablation of the bone marrow of said patient.

2. Use of a tubulin interacting compound for the preparation of a pharmaceutical composition for the treatment of cancer diseases and/or for the palliation of pain associated with cancer diseases, comprising the administration of the tubulin interacting compound in combination with a bone-localising radiopharmaceutical to the patient in an effective amount that will not cause any substantial ablation of the bone marrow of said patient.

3. Use according to claim 1 or 2, wherein the tubulin interacting compound is administered before and/or after the administration of the bone-localising radiopharmaceutical.

4. Use according to any of claims 1 to 3, wherein said effective amount is effective for bone pain relief in said patient.

5. Use according to any of claims 1 to 4, wherein said cancerous disease is selected from the group of cancer diseases, comprising multiple myeloma, leukaemia, lymphoma, breast cancer, prostate cancer, gynecologic cancer, gastric cancer ovarian cancer, lung cancer and/or renal cell carcinoma.

6. Use according to any of claims 1 to 5, wherein said cancerous disease is a cancer with bone metastasis, such as metastatic breast cancer or metastatic prostate cancer.

7. Use according to any of claims 1 to 6, wherein said cancerous disease is hormone-refractory prostate cancer (HRPC).

8. Use according to any of claims 1 to 7, wherein said bone-localising radiopharmaceutical is selected from the group consisting of the radionuclides ¹⁷⁷Lu, ¹⁷⁵Yb, ¹⁸⁶Re, ¹⁸⁸Re, ⁹⁰Y, ¹¹⁷mSn, ¹⁵³Sm, ¹⁶⁶Ho or ¹⁵⁹Gd that are complexed with a bone-localising chelating agent selected from the group consisting of polyaminocarboxylic acids and aminophosphonic acids, in particular samarium Sm 153 lexidronam, and ⁸⁹Sr-chloride.

9. Use according to claim 8, wherein said aminophosphonic acids are selected from the group consisting of EDTMP, DOTMP, DTPMP, HEEDTMP, NTMP, HEDP, HMDP, MDP, and TTHMP, and combinations thereof, and wherein said polyaminocarboxylic acids are selected from the group consisting of DTPA, EDTA, and DOTA, and combinations thereof.

10. Use according to any of claims 1 to 9, wherein said bone-localising radiopharmaceutical is administered at a dose of between about 0.005 to 5.0 mCi/kg, and preferably administered at a dose of 1.0 mCi/kg.

11. Use according to any of claims 1 to 10, wherein said tubulin interacting compound is administered at a dose of between 20 to 100 mg/m²/week, and preferably administered at a dose of approximately between 30 to 70 mg/m²/week.

12. Use according to any of claims 1 to 11, wherein said tubulin interacting compound is interacting directly and/or indirectly and is a chemotherapeutic agent selected from the group comprising vinca alkaloids, such as vincristine, vinblastine, vindesine, vinorelbine, taxanes, such as paclitaxel, docetaxel, epothilone A or B, and the like.

13. Use according to claim 12, wherein the chemotherapeutic agent is Taxol and is administered in a dose of approx. 70 mg/m²/week or is Taxotere which is administered in a dose of approx. 25-40 mg/m²/week.

14. Use according to any of claims 1 to 13, wherein the administration of said bone-localising radiopharmaceutical is performed in a time period ranging from about 1 to 30 days, and preferably 1 to 7 days, before and/or after the administration of said tubulin interacting compound.

15. Use according to any of claims 1 to 14, wherein said bone-localising radiopharmaceutical is administered in at least 8 week-intervals, preferably 16 week-intervals, during the course of a weekly administration of said tubulin interacting compound.

16. Use according to any of claims 1 to 15, wherein said bone-localising radiopharmaceutical is administered in at least 8 week-intervals, preferably 16 week-intervals, during the course of an administration every three weeks of said tubulin interacting compound.

17. Use according to any of claims 1 to 16, further comprising the administration of estramustine phosphate sodium, which is preferably administered weekly at a dose of between approximately 100 to 400mg, more preferably at approximately 280 mg twice a day.

18. Use according to any of claims 1 to 17, wherein said administration is intravenous (i.v.), orally (p.o.), and/or subcutaneously.

19. Use according to any of claims 1 to 18, wherein said administration is in a bolus dose or in several doses.

20. Kit of parts for the treatment of a cancer disease and/or for the palliation of pain associated with cancer diseases, said kit comprising the following components:
a) a pharmaceutically effective amount of a bone-localising radiopharmaceutical,
b) a pharmaceutically effective amount of a tubulin interacting compound, and
c) optionally, suitable pharmaceutically acceptable buffers and/or diluents,
wherein the amount of the bone-localising radiopharmaceutical comprises an effective amount of said bone-localising radiopharmaceutical that will not cause any substantial ablation of the bone marrow when applied to a patient.

21. Kit according to claim 20, wherein said bone-localising radiopharmaceutical is selected from the group consisting of the radionuclides ¹⁷⁷Lu, ¹⁷⁵Yb, ¹⁸⁶Re, ¹⁸⁸Re, ⁹⁰Y, ¹¹⁷mSn, ¹⁵³Sm, ¹⁶⁶Ho or ¹⁵⁹Gd that are complexed with a bone-localising chelating agent selected from the group consisting of polyaminocarboxylic acids and aminophosphonic acids, in particular samarium Sm 153 lexidronam, and ⁸⁹Sr-chloride.

22. Kit according to claim 20 or 21, wherein said aminophosphonic acids are selected from the group consisting of EDTMP, DOTMP, DTPMP, HEEDTMP, NTMP, HEDP, HMDP, MDP, and TTHMP, and combinations thereof, and wherein said polyaminocarboxylic acids are selected from the group consisting DTPA, EDTA, and DOTA, and combinations thereof.

23. Kit according to any of claims 20 to 22, wherein said bone-localising radiopharmaceutical is suitable for the administration at a dose of between about 0.005 to 5.0 mCi/kg, preferably at a dose of 1.0 mCi/kg.

24. Kit according to any of claims 20 to 23, wherein said tubulin interacting compound is suitable for the administration at a dose of between 20 to 100 mg/m²/week, and preferably administered at a dose of approximately between 30 to 70 mg/m²/week.

25. Kit according to any of claims 20 to 24, wherein said tubulin interacting compound is suitable for administration before or after the administration of said bone-localising radiopharmaceutical.

26. Kit according to any of claims 20 to 25, wherein said tubulin interacting compound is a chemotherapeutic agent selected from the group comprising vinca alkaloids, such as vincristine, vinblastine, vindesine, vinorelbine, taxanes, such as paclitaxel, docetaxel, epothilone A or B, and the like.

27. Kit according to claim 26, wherein the chemotherapeutic agent is Taxol and is suitable for administration in a dose of approx. 70 mg/m²/week or is Taxotere which is suitable for administration in a dose of approx. 25-40 mg/m²/week.

28. Kit according to any of claims 20 to 27, wherein said bone-localising radiopharmaceutical is suitable for administration in a time period ranging from about 1 to 30 days, and preferably 1 to 7 days, before and/or after the administration of said tubulin interacting compound.

29. Kit according to any of claims 20 to 28, wherein said bone-localising radiopharmaceutical is suitable for administration in at least 8 week-intervals, preferably 16 week-intervals, during the course of a weekly administration of said tubulin interacting compound.

30. Use of a kit according to any of claims 20 to 29, wherein said cancerous disease is selected from the group of cancer diseases, comprising multiple myeloma, leukaemia, lymphoma, breast cancer, ovarian cancer, lung cancer, renal cell carcinoma, and/or for bone pain relief in said patient.

31. Use of a kit according to any of claims 20 to 30, wherein the cancer disease is hormone-refractory prostate cancer (HRPC) and/or is a cancer with bone metastasis, such as metastatic breast cancer or metastatic prostate cancer.

32. Use of a kit according to any of claims 20 to 30, wherein said administration is intravenous (i.v.), orally (p.o.), and/or subcutaneously.

33. Use of a kit according to any of claims 20 to 30, wherein said administration is in a bolus dose or in several doses.
